# EUROPÄISCHE PATENTSCHRIFT

(11) **EP 2 756 828 B1**
(45) Veröffentlichungstag und Bekanntmachung des Hinweises auf die Patenterteilung: **27.02.2019**
(21) Anmeldenummer: 13000224.9
(22) Anmeldetag: 16.01.2013
(51) Int. Cl.: A61F 9/008

(54) **Ophthalmologische Vorrichtung zur Behandlung von Augengewebe**
Ophthalmologic apparatus for the treatment of eye tissue
Dispositif ophtalmologique destiné à traiter les tissus oculaires

(43) Veröffentlichungstag der Anmeldung: 23.07.2014
(73) Patentinhaber: Ziemer Ophthalmic Systems AG, 2562 Port (CH)
(72) Erfinder: Rathjen, Christian, 28197 Bremen (DE)
(74) Vertreter: Rentsch Partner AG

(56) Entgegenhaltungen:
- EP-A1- 1 584 310
- WO-A1-2011/091326
- WO-A1-2012/178054
- WO-A2-2011/017000
- US-A1- 2011 245 817
- US-A1- 2012 271 286

## Beschreibung

### Technisches Gebiet

Die vorliegende Erfindung betrifft eine ophthalmologische Vorrichtung zur Behandlung von Augengewebe mit Laserpulsen. Die vorliegende Erfindung betrifft insbesondere eine ophthalmologische Vorrichtung zur Behandlung von Augengewebe mit Laserpulsen, welche ein Scannersystem zum dynamischen Ablenken der Laserpulse und eine Projektionsoptik zur fokussierten Projektion der Laserpulse umfasst.

### Stand der Technik

Bei der Bearbeitung von Augengewebe mit gepulsten Laserstrahlen werden Fokussiervorrichtungen eingesetzt, um den Fokussierbereich zu verstellen. Insbesondere bei ophthalmologischen Lasersystemen mit erweiterten Anwendungsbereichen, die neben der Behandlung von Augengewebe der Hornhaut über die Hornhaut hinausreichende Fokuspositionen ermöglichen sollen, z.B. für die Linsenchirurgie oder die Behandlung anderer hinter der Hornhaut liegender Augenbereiche, werden Fokussiervorrichtungen im Strahlengang zwischen der Laserquelle und der Projektionsoptik vorgesehen, um den Fokussierbereich zu erweitern respektive zu verstellen. Diese Fokussiervorrichtungen führen im optischen Pfad Zoombewegungen respektive Zoomfunktionen zum Verstellen der Fokustiefe aus, so dass die fokussierte Projektion in Projektionsrichtung verschoben wird. Die Zoombewegungen respektive Zoomfunktionen umfassen eine Verschiebung eines oder mehrerer optischer Elemente entlang der optischen Achse eines Systems. Allgemein betrachtet kann eine Zoomaktion durch verschiebbare Linsen oder Spiegel aber auch durch andere optische Elemente ausgeführt werden, beispielsweise durch deformierbare Linsen, deformierbare Spiegel, SLM-Systeme (Spatial Light Modulator) oder Systeme mit dynamischer Modulation des Brechungsindex. Somit gelten als Zoomsysteme allgemein optische Systeme, die ihre Brennweite verstellen. Abhängig von der technischen Umsetzung werden dazu unterschiedlich viele Elemente benötigt. Die Fokusposition wird somit durch die Zoomeinstellung des Zoomsystems bestimmt, wobei die Zoomeinstellung je nach technischer Umsetzung durch Zoombewegungen von Linsen und/oder Spiegeln respektive Zoomaktionen oder Zoomfunktionen zur Einstellung von deformierbaren Linsen, deformierbaren Spiegeln, SLM-Systemen respektive Brechungsindexmodulatoren bestimmt wird.

In WO2011/017000 wird ein optisches System mit einem Scannersystem für die ophthalmologische Laserchirurgie beschrieben, welches ein Zoomsystem umfasst, das einen Tiefen- oder z-Scan über den Bereich der Hornhaut hinaus bis in die Tiefe der Augenlinse ermöglicht, wobei die Fokusposition über einen ausgedehnten Bereich von der Hornhaut bis in die Augenlinse verstellt wird. Im optischen System gemäss WO2011/017000 wird die Lage der virtuellen Eintrittspupille des Zoomsystems durch die beim Tiefen- oder z-Scan ausgeführten Zoomaktionen verändert. Dabei werden abhängig von der aktuellen Einstellung des Zoomsystems vergrösserte Distanzen des Scannersystems zur virtuellen Eintrittspupille des Zoomsystems verursacht. Dies hat zu Folge, dass das Zoomsystem im Durchmesser grösser und damit teurer ausgelegt werden muss (die Kosten steigen mindestens quadratisch mit dem Durchmesser). Weiterhin müssen grössere Scannersysteme, z.B. grössere Spiegel, eingesetzt werden oder andere Massnahmen getroffen werden, damit die vom Scannersystem abgelenkten Laserstrahlen uneingeschränkt in die Eintrittspupille des Zoomsystems eintreten können. Gemäss WO2011/017000 werden dazu zwei zusätzliche Scannachsen vorgeschlagen, die den gescannten Strahl wieder so auf die optische Achse ablenken, dass in jeder Zoomposition, der abgelenkte Laserstrahl die optische Achsen an dem Ort schneidet, wo sich die aktuelle Eintritts-Pupille des Zoom-Systems befindet. Mit dem Zusammenwirken der vier Scanner-Achsen kann einerseits der Ablenkungswinkel (Scannwinkel) in zwei Raumrichtungen eingestellt werden und andererseits auch der Ort des Schnittpunkts des abgelenkten Laserstrahls mit der optischen Achse bestimmt werden. Der Vorteil eines kompakteren optischen Systems wird hierbei jedoch mit dem Nachteil eines deutlich erhöhten Steuerungsaufwands und zusätzlichen Scannersystemen erkauft.

### Darstellung der Erfindung

Es ist eine Aufgabe der vorliegenden Erfindung eine ophthalmologische Vorrichtung zur Behandlung von Augengewebe mit Laserpulsen vorzuschlagen, welche zumindest einige Nachteile der bekannten Systeme nicht aufweist. Es ist insbesondere eine Aufgabe der vorliegenden Erfindung eine ophthalmologische Vorrichtung zur Behandlung von Augengewebe mit Laserpulsen vorzuschlagen, welche ein Verstellen der Fokusposition der Laserpulse in Projektionsrichtung ermöglicht, ohne dafür vergrösserte und/oder zusätzliche Scannerspiegel vorsehen zu müssen bzw. den Durchmesser und die Grösse des Zoomsystems zu minimieren.

Gemäss der vorliegenden Erfindung werden diese Ziele durch die Merkmale der unabhängigen Ansprüche erreicht. Weitere vorteilhafte Ausführungsformen gehen ausserdem aus den abhängigen Ansprüchen und der Beschreibung hervor.

Eine ophthalmologische Vorrichtung zur Behandlung von Augengewebe mit Laserpulsen umfasst ein Scannersystem zum dynamischen Ablenken der Laserpulse, eine Projektionsoptik zur fokussierten Projektion der Laserpulse, und ein Zoomsystem, das zwischen dem Scannersystem und der Projektionsoptik angeordnet ist und das eingerichtet ist, in verschiedenen Zoomeinstellungen die fokussierte Projektion der Laserpulse in Projektionsrichtung zu verstellen.

Die oben genannten Ziele werden durch die vorliegende Erfindung insbesondere dadurch erreicht, dass die ophthalmologische Vorrichtung überdies eine Verschiebevorrichtung umfasst, die eingerichtet ist, das Scannersystem abhängig von der Zoomeinstellung des Zoomsystems zu verschieben.

Eine einfache Verschiebebewegung des Scannersystems, die nur von der Zoomeinstellung, also beispielsweise von der Zoombewegung, abhängig ist, ermöglicht eine bedeutend einfachere Anbindung des Scannersystems an das Zoomsystem, als die im Stand der Technik beschriebene Lösung einer synchronisierten Bewegungen von vier Spiegelachsen. Gegenüber dem Stand der Technik können entsprechende Scannersysteme für zwei Spiegelachsen eingespart werden, was die thermische Belastung der optischen Systeme durch die Scannantriebe reduziert, und es muss keine Steuerung mit Regelalgorithmen für ein komplexes, vierachsiges Scannersystem bereitgestellt werden Überdies können auch die Gerätekosten insgesamt reduziert werden und durch die ermöglichte Verwendung kleiner Spiegel eine höhere Dynamik (Geschwindigkeit) erzielt werden

In einer bevorzugten Ausführungsvariante ist die Verschiebevorrichtung eingerichtet, das Scannersystem entlang einer optischen Achse des Zoomsystems zu verschieben. Vorzugsweise umfasst die Verschiebevorrichtung einen Antrieb zum Verschieben des Scannersystems.

In einer weiteren Ausführungsvariante ist die Verschiebevorrichtung eingerichtet, das Scannersystem synchronisiert mit der Zoomeinstellung des Zoomsystems zu verschieben.

In einer Ausführungsvariante ist die Verschiebevorrichtung eingerichtet, das Scannersystem synchronisiert mit der durch das Zoomsystem bewirkten Verstellung der fokussierten Projektion zu verschieben.

In einer weiteren Ausführungsvariante ist die Verschiebevorrichtung eingerichtet, das Scannersystem abhängig von der Position einer virtuellen Eintrittspupille des Zoomsystems zu verschieben. In einer Ausführungsvariante ist die Verschiebevorrichtung eingerichtet, das Scannersystem entlang einer optischen Achse des Zoomsystems an eine definierte Lage bezüglich der virtuellen Eintrittspupille des Zoomsystems zu verschieben. Das gesamte Scannersystem kann somit entlang der optischen Achse mit einer einfachen Translation verschoben werden, um die Ablenkspiegel möglichst dicht an oder in die virtuelle Eintrittspupille des Zoomsystems zu positionieren.

In einer weiteren Ausführungsvariante umfasst die ophthalmologische Vorrichtung ein Steuermodul, das eingerichtet ist, abhängig von Steuersignalen für das Zoomsystem oder von Feedbacksignalen vom Zoomsystem, Positionierungssignale zur Steuerung eines für die Verschiebung des Scannersystems vorgesehenen Antriebs zu erzeugen.

In einer Ausführungsvariante umfasst das Scannersystem einen bezüglich des Scannersystems starren Spiegel, der bei einer Verschiebung des Scannersystems mitbewegt wird und eingerichtet ist, die Laserpulse von einer Laserquelle auf einen beweglichen Ablenkspiegel des Scannersystems zu lenken.

In einer weiteren Ausführungsvariante umfasst die ophthalmologische Vorrichtung ein zwischen das Scannersystem und das Zoomsystem geschaltetes optisches Übertragungssystem, das eingerichtet ist, die vom Scannersystem abgelenkten Laserpulse an das Zoomsystem zu übertragen. Das optische Übertragungssystem ist beispielsweise in einem mechanischen Trägersystem angeordnet, über welches das Scannersystem und das Zoomsystem mechanisch miteinander verbunden sind.

In einer Ausführungsvariante umfasst die ophthalmologische Vorrichtung ein zwischen das Zoomsystem und die Projektionsoptik geschaltetes optisches Übertragungssystem, das eingerichtet ist, die Laserpulse vom Zoomsystem an die Projektionsoptik zu übertragen. Das optische Übertragungssystem ist beispielsweise in einem mechanischen Trägersystem angeordnet, über welches die Projektionsoptik und das Zoomsystem mechanisch miteinander verbunden sind.

In einer weiteren Ausführungsvariante umfasst die ophthalmologische Vorrichtung ein Kompensationssystem, das eingerichtet ist, Aberrationen des Zoomsystems abhängig von Verschiebung und Ablenkungswinkel des Scannersystems optisch zu kompensieren.

Die ophthalmologische Vorrichtung umfasst beispielsweise eine Laserquelle, die eingerichtet ist, Femtosekundenlaserpulse zu erzeugen. Das Scannersystem umfasst mindestens einen beweglichen Spiegel zum dynamischen Ablenken der Laserpulse. Die Projektionsoptik ist eingerichtet, die Laserpulse für eine Auflösung von Augengewebe fokussiert ins Augengewebe zu projizieren.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1:: zeigt schematisch im Querschnitt eine ophthalmologische Vorrichtung gemäss dem Stand der Technik zur Behandlung von Augengewebe mit Laserpulsen, welche ein Scannersystem und ein Zoomsystem aufweist.
- Figur 2:: zeigt schematisch im Querschnitt eine ophthalmologische Vorrichtung zur Behandlung von Augengewebe mit Laserpulsen, welche eine Verschiebevorrichtung zum Verschieben des Scannersystems abhängig von Einstellungen des Zoomsystems aufweist.
- Figuren 3 bis 7:: zeigen schematisch im Querschnitt verschiedene Ausführungsformen einer ophthalmologischen Vorrichtung zur Behandlung von Augengewebe mit Laserpulsen, welche eine Verschiebevorrichtung zum Verschieben des Scannersystems abhängig von Einstellungen des Zoomsystems aufweist.

### Wege zur Ausführung der Erfindung

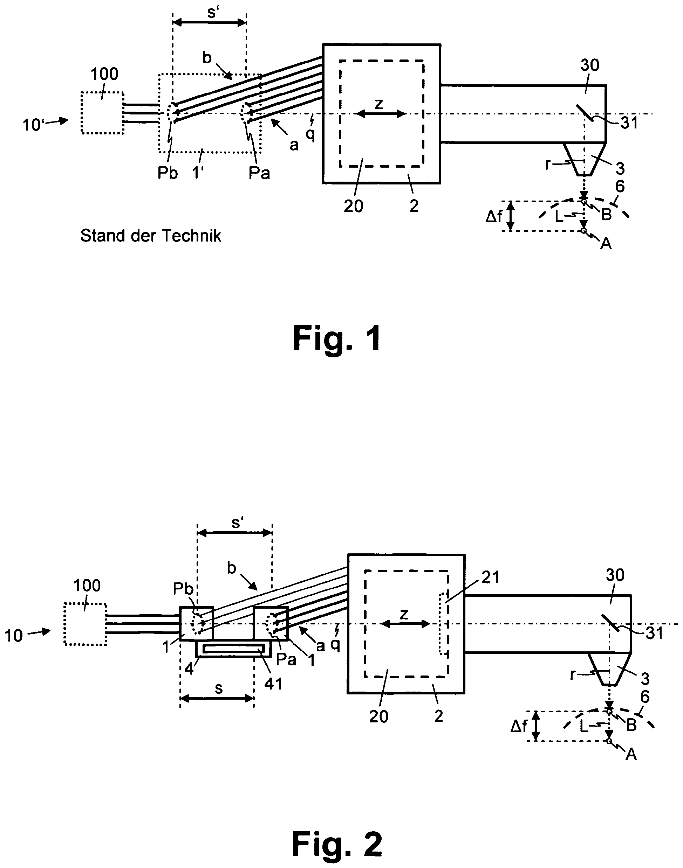

In der Figur 1 bezieht sich das Bezugszeichen 10' auf eine ophthalmologische Vorrichtung gemäss dem Stand der Technik zur Behandlung von Augengewebe 6 mit Laserpulsen L. Die ophthalmologische Vorrichtung 10' umfasst eine Laserquelle 100, eine Projektionsoptik 3 zur fokussierten Projektion der Laserpulse L, ein Scannersystem 1' zum dynamischen Ablenken der Laserpulse L und eine Fokussiervorrichtung 2 mit einem optischen Zoomsystem 20, das im Strahlengang zwischen der Projektionsoptik 3 und dem Scannersystem 1' angeordnet ist und das eingerichtet ist, die fokussierte Projektion der Laserpulse L, abhängig von der schematisch dargestellten Zoomeinstellung z des Zoomsystems 20, in Projektionsrichtung r zu verstellen, um verschiedene Fokustiefen A, B einzustellen, wie mit der Fokusverstellung Δf angezeigt wird.

Das Zoomsystem 20 umfasst beispielsweise mehrere (typisch zwei aber auch drei oder vier, wenn neben der Fokusposition noch andere optische Parameter wie Strahldivergenz oder Fokusdurchmesser eingestellt werden sollen und/oder Aberrationen kompensiert werden sollen) auf der optischen Achse q verschiebbare Linsen und/oder Linsengruppen, welche eingerichtet sind, durch Zoombewegungen die Zoomeinstellung z des Zoomsystems 20 zu verändern und zu bestimmen, um dadurch die fokussierte Projektion der Laserpulse L in Projektionsrichtung r zu verstellen und eine Fokusverstellung Δf für verschiedene Fokustiefen A, B zu erzeugen. Wie eingangs erwähnt wurde, kann das Zoomsystem 20 in alternativen Ausführungsvarianten neben oder statt verschiebbaren Linsen auch andere optische Elemente umfassen, beispielsweise Spiegel, deformierbare Linsen, deformierbare Spiegel, SLM-Systeme (Spatial Light Modulator) oder Systeme mit dynamischer Modulation des Brechungsindex, um mittels entsprechender Zoomaktionen oder Zoomfunktionen die Zoomeinstellung z des Zoomsystems 20 zu verändern und zu bestimmen.

Wie in den Figuren 1 bis 7 dargestellt ist, ist das Zoomsystem 20 zwischen der Projektionsoptik 3 und dem Scannersystem 1,1' angeordnet. Der Fachmann wird jedoch verstehen, dass abhängig von der Ausführungsvariante das Zoomsystem 20 auch Teile aufweisen kann, z.B. Linsen und/oder Linsengruppen, die dem Scannersystem 1, 1' vorgeschaltet sind, also auf dem optischen Pfad näher zur Laserquelle 100 (upstream) angeordnet sind.

Das Zoomsystem 20 weist eine veränderliche Eintrittspupille Pa, Pb auf. Bei der Eintrittspupille Pa, Pb eines optischen Systems handelt es sich um eine gedachte, also virtuelle, Blende, welche die Strahlen des optischen Systems begrenzt. Wie aus der Figur 1 ersichtlich ist, bewirkt die Verstellung der fokussierten Projektion der Laserpulse L durch das Zoomsystem 20 neben der Fokusverstellung Δf auch eine Verschiebung s' der Position der (virtuellen) Eintrittspupille Pa, Pb des Zoomsystems 20 entlang der optischen Achse q des Zoomsystems 20. Dadurch vergrössert sich der Abstand vom Scannersystem 1' zum Zoomsystem 20, was (im dargestellten Beispiel) eine Vergrösserung des Durchmessers des Zoomsystems 20 erforderlich macht und möglicherweise auch eine grössere Scannerapertur des Scannersystems 1' bedingt (andernfalls müsste der Scannwinkel verkleinert werden). Allgemein gilt, dass der Durchmesser des Zoomsystems 20 vergrössert werden muss, wenn die Scannerapertur nicht mit der Eintrittspupille des Zoomsystems 20 zusammenfällt (d.h. bei gleichem Durchmesser nicht am selben Ort liegt). Ansonsten wird der gescannte Strahl an physikalischen Blenden im Zoomsystem 20 abgeschnitten, was auch unter dem Begriff Vignettierung bekannt ist. Die Strahlverläufe in Figur 1 zeigen schematisch nur die nutzbaren Strahlquerschnitte. Bei der Verwendung eines Scannspiegels (mit Strahleinkopplung von der Seite) wird nachteilig auch nur ein Teil der eingestrahlten Laserleistung übertragen. Nicht dargestellt in Figur 1 wurde der Effekt, dass sich In Abhängigkeit vom Zoomsystem 20 auch die Grösse der Eintrittspupille Pa, Pb verändern kann. Die hier ausgeführten Zusammenhänge zeigen, dass eine optimale Auslegung von Scanner und Zoom mit dem Ziel sowohl die Scanneraperturen als auch den Durchmesser des Zoomsystems 20 zu minimieren bei der vom Stand der Technik bekannten Verwendung von zwei Scann-Achsen nicht realisierbar ist. Sowohl eine Vergrösserung des Zoomsystems 20 als auch eine Vergrösserung von Ablenkspiegeln sind mit erhöhten Kosten und den bereits eingangs beschriebenen Nachteilen verbunden.

In den Figuren 2 bis 7 bezieht sich das Bezugszeichen 10 auf eine ophthalmologische Vorrichtung zur Behandlung von Augengewebe 6 mit Laserpulsen L, welche im Unterschied zum Stand der Technik zusätzlich zu Laserquelle 100, Projektionsoptik 3, Scannersystem 1 und Fokussiervorrichtung 2 mit optischem Zoomsystem 20 eine Verschiebevorrichtung 4 aufweist, die eingerichtet ist, das Scannersystem 1 entlang der optischen Achse q des Zoomsystems 20 zu verschieben, wie durch die mit s bezeichnete Verschiebung (Translationsbewegung) angedeutet wird.

Das Scannersystem 1 umfasst einen oder mehrere Ablenkeinrichtungen, z.B. bewegliche Ablenkspiegel, welche eingerichtet sind, die Laserpulse L dynamisch in mindestens eine Ablenkrichtung abzulenken und dadurch den durch die Laserpulse L gebildeten Laserstrahl durch Verändern des Ablenkungswinkels (Scannwinkel bezüglich der optischen Achse) entlang mindestens einer Abtastrichtung (Scannrichtung) zu führen. Abhängig von der Ausführungsvariante umfasst das Scannersystem 1 einen oder mehrere Galvanoscanner, Polygonscanner, Resonanzscanner, AOM-Scanner (Akustooptischer Modulator), EOM-Scanner (Elektrooptischer Modulator) und/oder SLM-Scanner (Spatial Light Modulator).

Die Laserquelle 100 ist eingerichtet zur Erzeugung eines mit Laserpulsen L, z.B. mit Femtosekundenlaserpulsen, gepulsten Laserstrahls.

Die Verschiebevorrichtung 4 ist insbesondere eingerichtet, das Scannersystem 1 abhängig (i) von der aktuellen Zoomeinstellung z des Zoomsystems 20 zu verschieben. Das heisst, die Verschiebevorrichtung 4 ist eingerichtet, das Scannersystem 1 gekoppelt an (i) die durch das Zoomsystem 20 ausgeführte Zoombewegung/Zoomfunktion, respektive (ii) die durch die Zoombewegung/Zoomfunktion bewirkte Verschiebung s der virtuellen Eintrittspupille Pa, Pb des Zoomsystems 20, respektive (iii) die durch die Zoombewegung/Zoomfunktion bewirkte Fokusverstellung Δf zu verschieben.

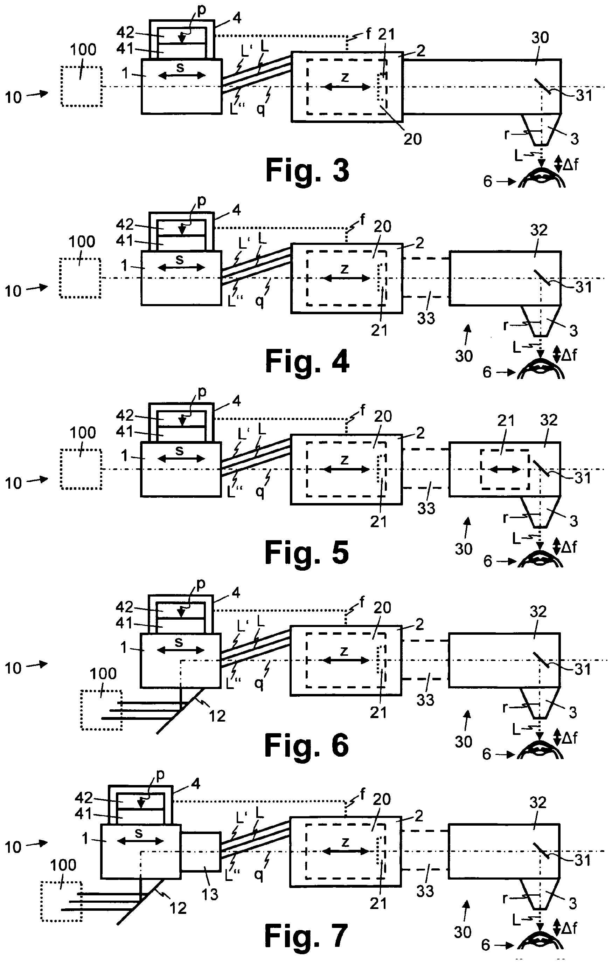

Wie in den Figuren 2 bis 7 schematisch dargestellt ist, umfasst die Verschiebevorrichtung 4 einen mit dem Scannersystem 1 gekoppelten Antrieb 41 zur Verschiebung des Scannersystems 1. Wie in den Figuren 3 bis 7 schematisch dargestellt ist, umfasst die Verschiebevorrichtung 4 überdies ein Steuermodul 42, das eingerichtet ist, den Antrieb 41 abhängig von der aktuellen Zoomeinstellung z des Zoomsystems 20, also abhängig von der Zoombewegung/Zoomfunktion des Zoomsystems 20, zu steuern. Das Steuermodul 42 umfasst einen oder mehrere Schaltkreise, beispielsweise einen Prozessor mit gespeichertem Computerprogrammcode, eine programmierte logische Schaltung und/oder eine elektronische Schaltung, welche eingerichtet sind, den Antrieb 41 zur Verschiebung des Scannersystems 1 abhängig von der aktuellen Zoomeinstellung z des Zoomsystems 20, also abhängig von der Zoombewegung/Zoomfunktion des Zoomsystems 20, zu steuern. Das Steuermodul 42 erzeugt dazu Positionierungssignale p, welche dem Antrieb 41 der Verschiebevorrichtung 4 zur Ausführung der Verschiebung s zugeführt werden. In einer Ausführungsvariante ist das Steuermodul 42 eingerichtet, die Positionierungssignale p für den Antrieb 41 abhängig von Fokussiersignalen f zu erzeugen, welche dem Zoomsystem 20 zum Bestimmen der aktuellen Zoomeinstellung z zugeführt werden, über die eine Fokusverstellung Δf zum Einstellen des gewünschten Projektionsfokus in Projektionsrichtung r erzielt wird. Dabei werden die Fokussiersignale f im Steuermodul 42 selber oder in einer anderen, vom Steuermodul 42 unabhängigen Steuereinheit, beispielsweise in der Fokussiervorrichtung 2, erzeugt. In einer alternativen Ausführung erzeugt das Zoomsystem 20 ein Feedbacksignal, das dem Steuermodul 42 laufend die aktuelle Zoomeinstellung z angibt. Das Steuermodul 42, steuert dementsprechend den Antrieb 41 abhängig vom Feedbacksignal, das die aktuelle Zoomeinstellung z angibt. Durch die Verschiebung s des Scannersystems 1 abhängig von der aktuellen Zoomeinstellung z des Zoomsystems 20 wird ermöglicht, die Position des Scannersystems 1 dynamisch und synchronisiert an die aktuelle Position der virtuellen Eintrittspupille Pa, Pb des Zoomsystems 20 anzupassen. Das Scannersystem 1 respektive dessen Ablenksiegel werden dabei laufend möglichst nah an die virtuelle Eintrittspupille Pa, Pb des Zoomsystems 20 heran positioniert, beispielsweise an eine Position mit einem fest definierten Distanzwert zur virtuellen Eintrittspupille Pa, Pb, oder direkt in die virtuelle Eintrittspupille Pa, Pb. Weiterhin kann die Scannerverschiebung auch in diskreten Schritten erfolgen. Im Falle der Vorderkammerchirurgie von Hornhaut und Linse müssten dazu mindestens zwei Scanner-Positionen angefahren werden, eine für die Hornhaut und einen für die Linse.

Das Zoomsystem 20 umfasst optional ein Kompensationssystem 21, das eingerichtet ist, Aberrationen des Zoomsystems 20 abhängig von Verschiebung s (und daher implizit auch von der aktuellen Zoomeinstellung z des Zoomsystems 20) und/oder Ablenkungswinkel(n) des Scannersystems 1 optisch zu kompensieren. Das Kompensationssystem 21 umfasst einen oder mehrere Schaltkreise, beispielsweise einen Prozessor mit gespeichertem Computerprogrammcode, eine programmierte logische Schaltung und/oder eine elektronische Schaltung, welche eingerichtet sind, das Zoomsystem 20 abhängig von der aktuellen Verschiebung s und dem (den) aktuellen Ablenkungswinkel(n) des Scannersystems 1 so zu steuern, dass Aberrationen des Zoomsystems 20 kompensiert werden, die bei der betreffenden Verschiebung s und dem (den) betreffenden Ablenkungswinkel(n) des Scannersystems 1 verursacht würden. In einer Ausführungsvariante umfasst das Kompensationssystem 21 dazu eine Kompensationstabelle oder Kompensationsfunktion, welche für verschiedene Verschiebungen s und Ablenkungswinkel des Scannersystems 1 jeweils Korrekturwerte oder kompensierte Fokussiersignale f zur Steuerung des Zoomsystems 20 angeben. In einer anderen Ausführungsvariante, wird die Verschiebung des Scannersystems s selbst dazu eingesetzt, um Aberrationen zu kompensieren.

Wie in den Figuren 1 bis 7 schematisch dargestellt ist umfasst die ophthalmologische Vorrichtung 10, 10' ein optisches Übertragungssystem 30, über welches die Laserpulse L von der Fokussiervorrichtung 2 respektive vom Zoomsystem 20 an die Projektionsoptik 3 geleitet werden.

Wie in den Figuren 4 bis 7 schematisch dargestellt ist, umfasst das optische Übertragungssystem 30 abhängig von der Ausführungsvariante ein optionales Tragsystem 33 und einen Applikationskopf 32, wobei die optischen Elemente des optischen Übertragungssystems 30 im Tragsystem 33 und/oder im Applikationskopf 32 angeordnet sind und die Projektionsoptik 3 am Applikationskopf 32 angebracht oder in den Applikationskopf 32 integriert ist. Die optischen Elemente des optischen Übertragungssystems 30 umfassen je nach Ausführungsvariante einen oder mehrere Spiegel zur Umlenkung des Laserstrahls respektive der Laserpulse L und eine oder mehrere Linsen, beispielsweise zur Erzeugung von einem oder mehreren Zwischenfoki im Strahlengang zwischen der Fokussiervorrichtung 2 respektive dem Zoomsystem 20 und der Projektionsoptik 3. In einer besonders einfachen Ausführungsform umfasst das optische Übertragungssystem 30 als einziges optisches Element einen Umlenkspiegel 31. In verschiedenen Ausführungsvarianten umfasst das Tragsystem 33 einen starren Tragarm, einen mehrgliedrigen, beweglichen Gelenkarm oder bloss ein Kopplungssystem zur festen oder entfernbaren Befestigung des Applikationskopfs 32 an der Fokussiervorrichtung 2.

Wie in der Figur 5 schematisch dargestellt ist, umfasst das optische Übertragungssystem 30 in einer Ausführungsvariante ein weiteres Zoomsystem 21, das eingerichtet ist, die fokussierte Projektion der Laserpulse L in Projektionsrichtung r zu verstellen, um verschiedene Fokustiefen einzustellen.

Wie in den Figuren 6 und 7 schematisch dargestellt ist, umfasst die ophthalmologische Vorrichtung 10 in einer Ausführungsvariante einen Umlenkspiegel 12, der fest am Scannersystem 1 angebracht und bezüglich des Scannersystems 1 unbeweglich ausgestaltet ist. Über diesen Umlenkspiegel 12 des Scannersystems 1 werden die Laserpulse L respektive der Laserstrahl von der Laserquelle 100 dem Scannersystem 1, insbesondere einem beweglichen Spiegel des Scannersystems 1, zugeführt.

Wie in der Figur 7 schematisch dargestellt ist, umfasst die ophthalmologische Vorrichtung 10 in einer Ausführungsvariante ein weiteres optisches Übertragungssystem 13, das im Strahlengang zwischen das Scannersystem 1 und die Fokussiervorrichtung 2 respektive das Zoomsystem 20 geschaltet ist. Die optischen Elemente dieses weiteren optischen Übertragungssystems 13 umfassen je nach Ausführungsvariante einen oder mehrere Spiegel zur Umlenkung des Laserstrahls respektive der Laserpulse L und/oder eine oder mehrere Linsen, beispielsweise zur Erzeugung von einem oder mehreren Zwischenfoki im Strahlengang zwischen dem Scannersystem 1 und der Fokussiervorrichtung 2 respektive dem Zoomsystem 20. Das weitere optische Übertragungssystem 13 ist beispielsweise in einem mechanischen Trägersystem angeordnet, welches das Scannersystem 1 und das Zoomsystem 20 mechanisch miteinander verbindet.

## Patentansprüche

1. Ophthalmologische Vorrichtung (10) zur Behandlung von Augengewebe (6) mit Laserpulsen (L), umfassend:
ein Scannersystem (1) zum dynamischen Ablenken der Laserpulse (L);
eine Projektionsoptik (3) zur fokussierten Projektion der Laserpulse (L),
ein Zoomsystem (20), das zwischen dem Scannersystem (1) und der Projektionsoptik (3) angeordnet ist und das eingerichtet ist, in verschiedenen Zoomeinstellungen (z) die fokussierte Projektion der Laserpulse (L) in Projektionsrichtung (r) zu verstellen,
**gekennzeichnet durch** eine Verschiebevorrichtung (4), die eingerichtet ist, das Scannersystem (1) abhängig von der Zoomeinstellung (z) des Zoomsystems (20) zu verschieben.

2. Ophthalmologische Vorrichtung (10) nach Anspruch 1, **dadurch gekennzeichnet, dass** die Verschiebevorrichtung (4) eingerichtet ist, das Scannersystem (1) entlang einer optischen Achse (q) des Zoomsystems (20) zu verschieben.

3. Ophthalmologische Vorrichtung (10) nach einem der Ansprüche 1 oder 2, **dadurch gekennzeichnet, dass** die Verschiebevorrichtung (4) eingerichtet ist, das Scannersystem (1) synchronisiert mit der Zoomeinstellung (z) des Zoomsystems (20) zu verschieben.

4. Ophthalmologische Vorrichtung (10) nach einem der Ansprüche 1 bis 3, **dadurch gekennzeichnet, dass** die Verschiebevorrichtung (4) eingerichtet ist, das Scannersystem (1) synchronisiert mit der durch das Zoomsystem (20) bewirkten Verstellung der fokussierten Projektion zu verschieben.

5. Ophthalmologische Vorrichtung (10) nach einem der Ansprüche 1 bis 4, **dadurch gekennzeichnet, dass** die Verschiebevorrichtung (4) eingerichtet ist, das Scannersystem (1) abhängig von der Position einer virtuellen Eintrittspupille (Pa, Pb) des Zoomsystems (20) zu verschieben.

6. Ophthalmologische Vorrichtung (10) nach einem der Ansprüche 1 bis 5, **dadurch gekennzeichnet, dass** die Verschiebevorrichtung (4) eingerichtet ist, das Scannersystem (1) entlang einer optischen Achse (q) des Zoomsystems (20) an eine definierte Lage bezüglich der virtuellen Eintrittspupille (Pa, Pb) des Zoomsystems (20) zu verschieben.

7. Ophthalmologische Vorrichtung (10) nach einem der Ansprüche 1 bis 6, **gekennzeichnet durch** ein Steuermodul (42), das eingerichtet ist, abhängig von Steuersignalen (f) für das Zoomsystem (20) oder von Feedbacksignalen vom Zoomsystem (20), Positionierungssignale (p) zur Steuerung eines für die Verschiebung des Scannersystems (1) vorgesehenen Antriebs (41) zu erzeugen.

8. Ophthalmologische Vorrichtung (10) nach einem der Ansprüche 1 bis 7, **dadurch gekennzeichnet, dass** das Scannersystem (1) einen bezüglich des Scannersystems (1) starren Spiegel (12) umfasst, der bei einer Verschiebung des Scannersystems (1) mitbewegt wird und eingerichtet ist, die Laserpulse (L) von einer Laserquelle (100) auf einen beweglichen Ablenkspiegel des Scannersystems (1) zu lenken.

9. Ophthalmologische Vorrichtung (10) nach einem der Ansprüche 1 bis 8, **gekennzeichnet durch** ein zwischen das Scannersystem (1) und das Zoomsystem (20) geschaltetes optisches Übertragungssystem (13), das eingerichtet ist, die vom Scannersystem (1) abgelenkten Laserpulse (L, L', L") an das Zoomsystem (20) zu übertragen.

10. Ophthalmologische Vorrichtung (10) nach Anspruch 9, **dadurch gekennzeichnet, dass** das optische Übertragungssystem (13) in einem mechanischen Trägersystem angeordnet ist, über welches das Scannersystem (1) und das Zoomsystem (20) mechanisch miteinander verbunden sind.

11. Ophthalmologische Vorrichtung (10) nach einem der Ansprüche 1 bis 10, **gekennzeichnet durch** ein zwischen das Zoomsystem (20) und die Projektionsoptik (3) geschaltetes optisches Übertragungssystem (30), das eingerichtet ist, die Laserpulse (L) vom Zoomsystem (20) an die Projektionsoptik (3) zu übertragen.

12. Ophthalmologische Vorrichtung (10) nach Anspruch 11, **dadurch gekennzeichnet, dass** das optische Übertragungssystem (30) in einem mechanischen Trägersystem angeordnet ist, über welches die Projektionsoptik (3) und das Zoomsystem (20) mechanisch miteinander verbunden sind.

13. Ophthalmologische Vorrichtung (10) nach einem der Ansprüche 1 bis 12, **dadurch gekennzeichnet, dass** die ophthalmologische Vorrichtung (10) eine Laserquelle (100) umfasst, die eingerichtet ist, Femtosekundenlaserpulse (L) zu erzeugen, dass das Scannersystem (1) mindestens einen beweglichen Spiegel zum dynamischen Ablenken der Laserpulse (L) umfasst, und dass die Projektionsoptik (3) eingerichtet ist, die Laserpulse (L) für eine Auflösung von Augengewebe (6) fokussiert ins Augengewebe (6) zu projizieren.

14. Ophthalmologische Vorrichtung (10) nach einem der Ansprüche 1 bis 13, **gekennzeichnet durch** ein Kompensationssystem (21), das eingerichtet ist, Aberrationen des Zoomsystems (20) abhängig von Verschiebung und Ablenkungswinkel des Scannersystems (1) optisch zu kompensieren.

15. Ophthalmologische Vorrichtung (10) nach einem der Ansprüche 1 bis 14, **dadurch gekennzeichnet, dass** die Verschiebevorrichtung (4) einen Antrieb (41) zum Verschieben des Scannersystems (1) umfasst.

## Claims

1. Ophthalmological device (10) for treating eye tissue (6) with laser pulses (L), comprising:
a scanner system (1) for dynamically deflecting the laser pulses (L);
a projection optical unit (3) for focused projection of the laser pulses (L),
a zoom system (20), which is arranged between the scanner system (1) and the projection optical unit (3) and which is configured to adjust the focused projection of the laser pulses (L) in the projection direction (r) in different zoom settings (z),
**characterized by** a displacement device (4), which is configured to displace the scanner system (1) depending on the zoom setting (z) of the zoom system (20).

2. Ophthalmological device (10) according to Claim 1, wherein the displacement device (4) is configured to displace the scanner system (1) along an optical axis (q) of the zoom system (20).

3. Ophthalmological device (10) according to either of Claims 1 or 2, wherein the displacement device (4) is configured to displace the scanner system (1) in a synchronized manner with the zoom setting (z) of the zoom system (20).

4. Ophthalmological device (10) according to one of Claims 1 to 3, wherein the displacement device (4) is configured to displace the scanner system (1) in a synchronized manner with the adjustment of the focused projection brought about by the zoom system (20).

5. Ophthalmological device (10) according to one of Claims 1 to 4, wherein the displacement device (4) is configured to displace the scanner system (1) depending on the position of a virtual entry pupil (Pa, Pb) of the zoom system (20).

6. Ophthalmological device (10) according to one of Claims 1 to 5, wherein the displacement device (4) is configured to displace the scanner system (1) along an optical axis (q) of the zoom system (20) to a defined position in respect of the virtual entry pupil (Pa, Pb) of the zoom system (20).

7. Ophthalmological device (10) according to one of Claims 1 to 6, **characterized by** a control module (42), which is configured to generate positioning signals (p) for controlling a drive (41) provided for displacing the scanner system (1), depending on control signals (f) for the zoom system (20) or on feedback signals from the zoom system (20).

8. Ophthalmological device (10) according to one of Claims 1 to 7, wherein the scanner system (1) comprises a mirror (12) which is rigid in relation to the scanner system (1), also moved in the case of a displacement of the scanner system (1) and configured to guide the laser pulses (L) from a laser source (100) to a movable deflection mirror of the scanner system (1).

9. Ophthalmological device (10) according to one of Claims 1 to 8, **characterized by** an optical transmission system (13), which is interposed between the scanner system (1) and the zoom system (20) and configured to transmit the laser pulses (L, L', L") deflected by the scanner system (1) to the zoom system (20).

10. Ophthalmological device (10) according to Claim 9, wherein the optical transmission system (13) is arranged in a mechanical support system, by means of which the scanner system (1) and the zoom system (20) are mechanically connected to one another.

11. Ophthalmological device (10) according to one of Claims 1 to 10, **characterized by** an optical transmission system (30), which is interposed between the zoom system (20) and the projection optical unit (3) and configured to transmit the laser pulses (L) from the zoom system (20) to the projection optical unit (3).

12. Ophthalmological device (10) according to Claim 11, wherein the optical transmission system (30) is arranged in a mechanical support system, by means of which the projection optical unit (3) and the zoom system (20) are mechanically connected to one another.

13. Ophthalmological device (10) according to one of Claims 1 to 12, wherein the ophthalmological device (10) comprises a laser source (100), which is configured to generate femtosecond laser pulses (L), wherein the scanner system (1) comprises at least one movable mirror for dynamically deflecting the laser pulses (L) and wherein the projection optical unit (3) is configured to project the laser pulses (L) into the eye tissue (6) in a focused manner in order to break down eye tissue (6).

14. Ophthalmological device (10) according to one of Claims 1 to 13, **characterized by** a compensation system (21), which is configured to optically compensate aberrations of the zoom system (20) depending on displacement and deflection angle of the scanner system (1).

15. Ophthalmological device (10) according to one of Claims 1 to 14, wherein the displacement device (4) comprises a drive (41) for displacing the scanner system (1).

## Revendications

1. Dispositif ophtalmologique (10) destiné au traitement de tissus oculaires (6) au moyen d'impulsions laser (L), comprenant :
un système de balayage (1) pour la déviation dynamique des impulsions laser (L) ;
une optique de projection (3) pour la projection focalisée des impulsions laser (L),
un système de zoom (20) qui est disposé entre le système de balayage (1) et l'optique de projection (3) et qui est conçu pour ajuster la projection focalisée des impulsions laser (L) dans la direction de projection (r) pour différents réglages du zoom (z),
**caractérisé par** un dispositif de déplacement (4) conçu pour déplacer le système de balayage (1) en fonction du réglage de zoom (z) du système de zoom (20).

2. Dispositif ophtalmologique (10) selon la revendication 1, **caractérisé en ce que** le dispositif de déplacement (4) est conçu pour déplacer le système de balayage (1) le long d'un axe optique (q) du système de zoom (20).

3. Dispositif ophtalmologique (10) selon l'une des revendications 1 ou 2, **caractérisé en ce que** le dispositif de déplacement (4) est conçu pour déplacer le système de balayage (1) en synchronisme avec le réglage de zoom (z) du système de zoom (20).

4. Dispositif ophtalmologique (10) selon l'une des revendications 1 à 3, **caractérisé en ce que** le dispositif de déplacement (4) est conçu pour déplacer le système de balayage (1) en synchronisme avec le réglage de la projection focalisée effectué par le système de zoom (20).

5. Dispositif ophtalmologique (10) selon l'une des revendications 1 à 4, **caractérisé en ce que** le dispositif de déplacement (4) est conçu pour déplacer le système de balayage (1) en fonction de la position d'une pupille d'entrée virtuelle (Pa, Pb) du système de zoom (20) .

6. Dispositif ophtalmologique (10) selon l'une des revendications 1 à 5, **caractérisé en ce que** le dispositif de déplacement (4) est conçu pour déplacer le système de balayage (1) le long d'un axe optique (q) du système de zoom (20) vers une position définie par rapport à la pupille d'entrée virtuelle (Pa, Pb) du système de zoom (20).

7. Dispositif ophtalmologique (10) selon l'une des revendications 1 à 6, **caractérisé par** un module de commande (42) conçu pour générer, en réponse à des signaux de commande (f) destinés au système de zoom (20) ou à des signaux de rétroaction provenant du système de zoom (20), des signaux de positionnement (p) destiné à commander un dispositif d'entraînement (41) prévu pour déplacer le système de balayage (1).

8. Dispositif ophtalmologique (10) selon l'une des revendications 1 à 7, **caractérisé en ce que** le système de balayage (1) comprend un miroir (12) rigide par rapport au système de balayage (1), qui est entraîné en association avec un déplacement du système de balayage (1) et est conçu pour diriger les impulsions laser (L) depuis une source laser (100) vers un miroir de déviation mobile du système de balayage (1).

9. Dispositif ophtalmologique (10) selon l'une des revendications 1 à 8, **caractérisé par** un système de transmission optique (13) qui est connecté entre le système de balayage (1) et le système de zoom (20) et qui est conçu pour transmettre les impulsions laser (L, L', L") déviées du système de balayage (1) au système de zoom (20) .

10. Dispositif ophtalmologique (10) selon la revendication 9, **caractérisé en ce que** le système de transmission optique (13) est disposé dans un système de support mécanique par l'intermédiaire duquel le système de balayage (1) et le système de zoom (20) sont reliés mécaniquement entre eux.

11. Dispositif ophtalmologique (10) selon l'une des revendications 1 à 10, **caractérisé par** un système de transmission optique (30) connecté entre le système de zoom (20) et l'optique de projection (3), qui est conçu pour transmettre les impulsions laser (L) du système de zoom (20) à l'optique de projection (3).

12. Dispositif ophtalmologique (10) selon la revendication 11, **caractérisé en ce que** le système de transmission optique (30) est disposé dans un système de support mécanique au moyen duquel l'optique de projection (3) et le système de zoom (20) sont reliés mécaniquement entre eux.

13. Dispositif ophtalmologique (10) selon l'une des revendications 1 à 12, **caractérisé en ce que** le dispositif ophtalmologique (10) comprend une source laser (100) conçue pour générer des impulsions laser femtosecondes (L), **en ce que** le système de balayage (1) comprend au moins un miroir mobile pour la déviation dynamique des impulsions laser (L), et **en ce que** l'optique de projection (3) est conçue pour projeter les impulsions laser (L) de manière focalisée dans les tissus oculaires (6) afin de désintégrer les tissus oculaires (6) .

14. Dispositif ophtalmologique (10) selon l'une des revendications 1 à 13, **caractérisé par** un système de compensation (21) conçu pour compenser optiquement les aberrations du système de zoom (20) en fonction du déplacement et de l'angle de déviation du système de balayage (1).

15. Dispositif ophtalmologique (10) selon l'une des revendications 1 à 14, **caractérisé en ce que** le dispositif de déplacement (4) comprend un dispositif d'entraînement (41) destiné à déplacer le système de balayage (1).
